(19) Europäisches Patentamt
European Patent Office
Office européen des brevets

(11) Veröffentlichungsnummer: **0 353 571 B1**

(12) **EUROPÄISCHE PATENTSCHRIFT**

(45) Veröffentlichungstag der Patentschrift: **12.10.94**

(21) Anmeldenummer: **89113498.3**

(22) Anmeldetag: **22.07.89**

(51) Int. Cl.[5]: **C07D 233/60**, A01N 43/50, C07D 233/68, C07D 233/70, C07D 233/54, C07D 231/56, C07D 231/16, C07D 231/12, C07D 249/02, C07D 235/06, C07D 235/08

(54) **Phenoxyalkylsubstituierte Heteroaromaten, Verfahren zu ihrer Herstellung und ihre Verwendung als Schädlingsbekämpfungsmittel.**

(30) Priorität: **29.07.88 DE 3825821**
**29.07.88 DE 3825822**

(43) Veröffentlichungstag der Anmeldung:
**07.02.90 Patentblatt 90/06**

(45) Bekanntmachung des Hinweises auf die Patenterteilung:
**12.10.94 Patentblatt 94/41**

(84) Benannte Vertragsstaaten:
**BE CH DE ES FR GB IT LI NL**

(56) Entgegenhaltungen:
**EP-A- 0 069 848** **EP-A- 0 132 606**
**EP-A- 0 203 798** **EP-A- 0 239 047**
**EP-A- 0 289 919** **DE-A- 2 516 331**

(73) Patentinhaber: **BASF Aktiengesellschaft**
**Carl-Bosch-Strasse 38**
**D-67063 Ludwigshafen (DE)**

(72) Erfinder: **Leyendecker, Joachim, Dr.**
**Stahlbuehlring 79**
**D-6802 Ladenburg (DE)**
Erfinder: **Neubauer, Hans-Juergen, Dr.**
**Mozartstrasse 6**
**D-4400 Muenster-Hiltrup (DE)**
Erfinder: **Kardorff, Uwe, Dr.**
**D 3,4**
**D-6800 Mannheim 1 (DE)**
Erfinder: **Kuenast, Christoph, Dr.**
**Salierstrasse 17**
**D-6701 Otterstadt (DE)**
Erfinder: **Krieg, Wolfgang, Dr.**
**Saarstrasse 17**
**D-6721 Weingarten (DE)**
Erfinder: **Hofmeister, Peter, Dr.**
**Bernard-Humblot-Strasse 12**
**D-6730 Neustadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 353 571 B1

## Beschreibung

Die vorliegende Erfindung betrifft neue phenoxyalkylsubstituierte Heteroaromaten der allgemeinen Formeln Ia und Ib

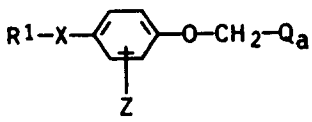

(Ia)

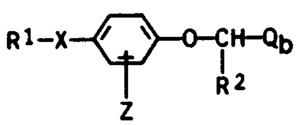

(Ib)

in denen die Substituenten die folgende Bedeutung haben:

| | |
|---|---|
| $R^1$ | $C_1$-$C_{12}$-Alkyl, $C_2$-$C_{12}$-Alkenyl, $C_2$-$C_{12}$-Alkinyl, $C_1$-$C_{12}$-Halogenalkyl, $C_2$-$C_{12}$-Halogenalkenyl, $C_2$-$C_{12}$-Halogenalkinyl, $C_3$-$C_{12}$-Alkoxyalkyl, $C_3$-$C_8$-Cycloalkyl, $C_4$-$C_{12}$-Cycloalkyl-alkyl, $C_3$-$C_8$-Halogencycloalkyl, $C_4$-$C_{12}$-Halogencycloalkyl-alkyl, $C_4$-$C_{12}$-Cycloalkyl-halogenalkyl oder $C_4$-$C_{12}$-Halogencycloalkyl-halogenalkyl; |
| X | O, S, $SO_2$; |
| Z | Wasserstoff, Halogen, $C_1$-$C_8$-Alkyl, $C_1$-$C_8$-Alkoxy, $C_1$-$C_4$-Halogenalkyl, $C_1$-$C_4$-Halogenalkoxy, $C_3$-$C_{10}$-Cycloalkyl, Nitro oder Cyano, im Fall des Heteroaromoten $Q_a$ und Wasserstoff, Halogen oder $C_1$-$C_4$-Alkyl im Fall des Heteroaromaten $Q_b$; |
| $R^2$ | Wasserstoff oder $C_1$-$C_4$-Alkyl; |
| $Q_a$ | ein gegebenenfalls substituierter Azolrest der Formel IIa-IIf |

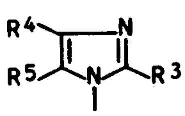

(IIa)

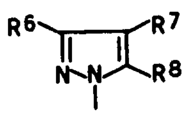

(IIb)

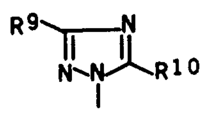

(IIc)

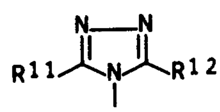

(IId)

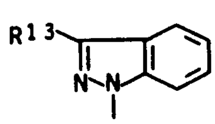

(IIe)

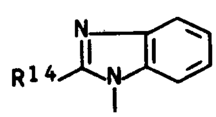

(IIf)

in denen

| | |
|---|---|
| $R^3$ bis $R^{14}$ | Wasserstoff, Halogen, $C_1$-$C_8$-Alkyl, $C_1$-$C_4$-Halogenalkyl, $C_1$-$C_4$-Alkoxy, $C_3$-$C_{10}$-Cycloalkyl, Phenyl, 1-Naphthyl und 2-Naphthyl, welches gegebenenfalls ein- bis dreifach durch Halogen, $C_1$-$C_8$-Alkoxy, $C_1$-$C_4$-Halogenalkyl oder $C_1$-$C_4$-Halogenalkoxy substituiert ist, |
| $Q_b$ | ein 5-Ring-Heteroaromat ausgewählt aus der Gruppe Thien-2-yl, Thien-3-yl, Thiazol-4-yl, Imidazol-5-yl, Pyrazol-3-yl, 1,3,4-Thiadiazol-2-yl, 1,3,4-Oxadiazol-2-yl, 1,2,4-Oxadiazol-3-yl, Isoxazol-5-yl und Isoxazol-3-yl, welcher gegebenenfalls ein- oder mehrfach mit Halogen, $C_1$-$C_8$-Alkyl, $C_2$-$C_8$-Alkenyl, $C_1$-$C_4$-Halogenalkyl, $C_1$-$C_8$-Alkoxy, $C_1$-$C_8$-Alkylthio, $C_2$-$C_8$-Alkoxyalkyl oder $C_3$-$C_{10}$-Cycloalkyl substituiert ist. |

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus EP-A-132 606 sind N-substituierte Azole als insektizide und akarizide Wirkstoffe bekannt. Die Wirkung dieser Verbindungen läßt jedoch zu wünschen übrig.

In EP-A 0 203 798 ist beispielsweise in 2-Stellung phenoxyethoxysubstituiertes 1,3-Thiazol der Struktur A

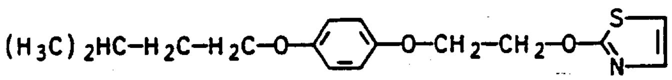

(A)

beschrieben. Phenoxymethylsubstituierte Heteroaromaten sind aus DE-A 25 16 331 und EP-A 239 047 bekannt. Die insektizide Wirkung dieser Verbindungen läßt ebenfalls zu wünschen übrig.

Der Erfindung lag daher die Aufgabe zugrunde, neue phenoxyalkylsubstituierte Heteroaromaten Ia und Ib mit verbesserter Wirkung bereitzustellen.

Demgemäß wurden die eingangs definierten neuen phenoxyalkylsubstituierten Heteroaromaten sowie Verfahren zu deren Herstellung gefunden. Ferner wurde gefunden, daß sich die Verbindungen Ia und Ib hervorragend zur Bekämpfung von Schädlingen eignen.

Die Verbindungen Ia und Ib sind nach folgenden Methoden erhältlich:

Die Umsetzung erfolgt beispielsweise zwischen einem Phenol III und einem N-Methylazol IVa oder einem Fünfringheteroaromaten IVb in Gegenwart einer Base im Temperaturbereich von (-20) bis 250 °C, vorzugsweise von 20 bis 120 °C, gemäß folgenden Reaktionsgleichungen:

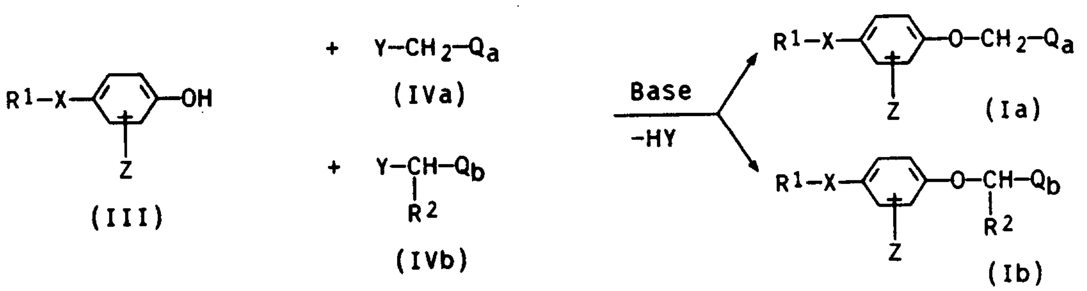


(III) (IVa) (IVb) (Ia) (Ib)

Die Phenole III sind zum Teil aus Houben/Weyl, Bd. VI, 3, Methoden der org. Chemie, Thieme Verlag, 1965, 49 ff und 85 ff bekannt, oder können nach den dort beschriebenen Methoden hergestellt werden.

Die N-Methylazole IVa sind zum Teil aus Heterocycles 24, 2233-2237 (1986) bekannt, oder können nach der dort beschriebenen Methode gemäß folgender Reaktionsgleichung hergestellt werden:

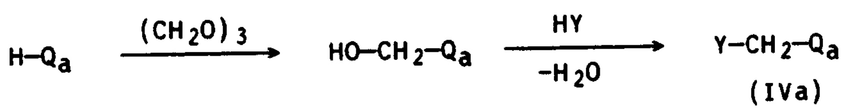


(IVa)

Die Heteroaromaten IVb sind entweder bekannt und teilweise kommerziell erhältlich oder lassen sich nach allgemein bekannten chemischen Verfahren herstellen. Verfahren zur Herstellung von Thiophenderivaten finden sich z.B. in Comprehensive Heterocyclic Chemistry, A.R.Katritzky und C.W.Rees, Vol. 4, S. 863 ff, Pergamon Press 1984; von Furanderivaten, z.B. in DE-A 35 14 384, DE-A 35 46 371 oder Advances in Heterocyclic Chemistry, Vol. 30, S. 167 ff, 1982; von Pyrrolderivaten z.B. in Comprehensive Heterocyclic Chemistry, A.R. Katritzky und C.W. Rees, Vol. 4, S. 313 ff, Pergamon Press, 1984, von Thiazolderivaten, Oxazolderivaten, Isothiazolderivaten, Thiadiazolderivaten und Oxadiazolderivaten z.B. in Comprehensive Heterocyclic Chemistry, R. Katritzky und W. Rees, Vol. 6, S. 131, 177, 235, 447, 365 ff, Pergamon Press, 1984; von Imidazolderivaten z.B. in Advances in Heterocyclic Chemistry, Vol. 27, S. 242 ff, 1980; von Pyrazolderivaten z.B. in Heteroaromatic Nitrogen Compounds, The Azoles, S. 31 ff, Cambridge University Press, 1976; von Triazolderivaten z.B. in Comprehensive Heterocyclic Chemistry, A.R. Katritzky und C.W. Rees, Vol. 5, S. 669 ff, Pergamon Press, 1984; von Isoxazolderivaten z.B. DE-A 25 49 962 und DE-A 27 54 832.

Man setzt normalerweise mindestens äquivalente Mengen einer Base zu III und/oder IVa bzw. IVb zu, kann aber die Base auch im Überschuß oder gegebenenfalls auch als Lösungsmittel verwenden.

Als Basen eignen sich beispielsweise Hydroxide von Alkali- und Erdalkalimetallen, wie Natriumhydroxid, Kaliumhydroxid und Calciumhydroxid, Alkoholate von Alkali- und Erdalkalimetallen, wie Natriummethylat, Natriumethylat, Calciummethanolat oder Kalium-tert.-butylat; Alkali- oder Erdalkalimetallhydride, wie Natriumhydrid, Kaliumhydrid oder Calciumhydrid; Alkali- oder Erdalkalicarbonate, wie Natriumcarbonat, Kaliumcarbonat oder Calciumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol und gegebenenfalls auch Alkyllithium-Verbindungen, wie n-Butyllithium.

Die Umsetzung wird zweckmäßigerweise in einem Lösungsmittel oder Verdünnungsmittel durchgeführt. Hierzu eignen sich beispielsweise aliphatische Kohlenwasserstoffe, wie n-Pentan, n-Hexan, das Hexan-Isomerengemisch und Petrolether; aromatische Kohlenwasserstoffe, wie Benzol, Toluol, die Xylole und deren Isomerengemische, Benzin; Alkohole, wie Methanol, Ethanol, n-Propanol und Isopropanol; Ether, wie Diethyl-, Di-n-Butylether, Methyl-tert.-butylether, Tetrahydrofuran und Dioxan; Ketone, wie Aceton, Methylethylketon und Methylisopropylketon; Nitrile, wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Anstelle des Phenols III plus Base kann auch das Phenolatanion von III direkt mit IVa bzw. IVb umgesetzt werden. In diesem Fall liegen die Reaktionstemperaturen bei (-20) bis 120 °C, vorzugsweise (-20) bis 80 °C.

Die Anionen der Phenole III sind in Form ihrer Metallsalze, wie dem Natrium oder Kaliumsalz, bekannt oder lassen sich in situ aus Phenolen III durch Umsetzung mit gängigen, z.B. in Houben/Weyl (s.o.) beschriebenen Metallierungsreagentien, wie Natriummethylat, Natriumethylat, Kalium-tert.-butylat, Natrium- bzw. Kaliumhydrid oder n-Butyllithium, generieren.

Die Umsetzung wird zweckmäßigerweise in einem Lösungsmittel oder Verdünnungsmittel durchgeführt. Hierzu eignen sich beispielsweise aliphatische Kohlenwasserstoffe wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe wie Benzol, Toluol, die Xylole und deren Isomerengemische und Benzin, Ether, wie Diethyl-, Di-n-Butylether, Methyl-tert.-butylether, Tetrahydrofuran und Dioxan; Ketone, wie Aceton, Methylethylketon und Methylisopropylketon; aprotische dipolare Lösungsmittel wie Dimethylformamid, Dimethylsulfoxid oder Pyridin. Auch Gemische dieser Stoffe können als Lösungs- und Verdünnungsmittel verwendet werden.

In allen Fallgestaltungen bedeutet der Rest Y eine übliche Abgangsgruppe, wie beispielsweise einen Sulfonsäurerest oder ein Halogen. Unter den Sulfonsäureresten sind Methansulfonyl, Trifluormethansulfonyl und p-Toluolsulfonyl, unter den Halogenen sind Chlor und Brom bevorzugt; besonders bevorzugt wird Chlor.

Zur Herstellung der erfindungsgemäßen Verbindungen Ia und Ib nach den vorstehend beschriebenen Methoden setzt man die Ausgangsstoffe üblicherweise in stöchiometrischem Verhältnis ein. Ein Überschuß des einen oder anderen Ausgangsstoffes kann in Einzelfällen aber durchaus vorteilhaft sein.

Die Umsetzungen verlaufen gewöhnlich oberhalb -20 °C mit ausreichenden Geschwindigkeiten. 120 °C müssen im allgemeinen nicht überschritten werden. Da sie in einigen Fällen unter Wärmeentwicklung verlaufen, kann es von Vorteil sein, Kühlmöglichkeiten vorzusehen.

Die Reaktionsgemische werden in üblicher Weise aufgearbeitet, z.B. durch Versetzen mit Wasser, Trennen der Phasen und Säulenchromatographie. Die neuen Verbindungen der Formeln Ia bzw. Ib fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formeln Ia bzw. Ib in kristalliner Form, so kann ihre Reinigung durch Umkristallisieren erfolgen.

Die Substituenten in Formel I haben im einzelnen folgende Bedeutungen:

$R^1$

- unverzweigtes oder verzweigtes $C_1$-$C_{12}$-Alkyl, bevorzugt $C_1$-$C_8$-Alkyl, besonders bevorzugt verzweigtes $C_3$-$C_8$-Alkyl, wie Isopropyl, Isobutyl, sec.-Butyl, tert.-Butyl, Isopentyl, 1-Methyl-butyl, Isohexyl, 1-Methyl-pentyl, Isoheptyl, 1-Methyl-hexyl, 1-Methyl-heptyl, 1,3-Dimethyl-butyl, 1,2-Dimethyl-butyl, 3-Methyl-pentyl, 4-Methyl-butyl, 1-Ethyl-propyl und 1-Ethyl-butyl;
- unverzweigtes oder verzweigtes $C_2$-$C_{12}$-Alkenyl, bevorzugt $C_2$-$C_8$-Alkenyl, besonders bevorzugt $C_3$-$C_6$-Alkenyl wie Allyl, 1-Methyl-allyl, 1,3-Dimethyl-but-2-enyl, 1-Methyl-but-2-enyl und But-3-en-1-yl;
- unverzweigtes oder verzweigtes $C_2$-$C_{12}$-Alkinyl, bevorzugt $C_2$-$C_8$-Alkinyl, besonders bevorzugt $C_2$-$C_4$-Alkinyl wie Ethinyl, Propinyl, Prop-2-inyl, 1-Methyl-prop-2-inyl, But-2-inyl und But-3-inyl;
- unverzweigtes oder verzweigtes $C_1$-$C_{12}$-Halogenalkyl, bevorzugt $C_1$-$C_4$-Halogenalkyl, besonders bevorzugt $C_1$-$C_4$-Fluor- und Chloralkyl wie Trifluormethyl, Difluormethyl, Fluormethyl, Trichlormethyl, Pentafluorethyl, 2,2,2-Trifluorethyl, 2,2,2-Trichlorethyl, 1-Fluormethyl-(2-fluor)-ethyl und 1-Chlormethyl-(2-chlor)-ethyl;

- unverzweigtes oder verzweigtes $C_2$-$C_{12}$-Halogenalkenyl, bevorzugt $C_2$-$C_4$-Halogenalkenyl, besonders bevorzugt $C_2$-$C_4$-Fluor- und Chloralkenyl wie 1,2,2-Trifluorethenyl, 1,2,2-Trichlorethenyl, 3,3-Difluorprop-2-enyl und 3,3-Dichlorprop-2-enyl;
- unverzweigtes oder verzweigtes $C_3$-$C_{12}$-Alkoxyalkyl, bevorzugt $C_3$-$C_9$-Alkoxyalkyl, besonders bevorzugt $C_1$-$C_4$-alkoxysubstituierte $C_1$-$C_5$-Alkoxyalkyl wie beispielsweise 2-Methoxyethyl, 2-Methoxypropyl, 3-Methoxyprop-2-yl, 3-Methoxybutyl, 4-Methoxybut-2-yl, 4-Methoxybut-3-yl, 5-Methoxypent-3-yl, 2-Ethoxyethyl, 2-Ethoxypropyl, 3-Ethoxypropyl, 3-Ethoxybutyl, 3-Ethoxybut-2-yl, 5-Ethoxypent-2-yl, 2-Propyloxyethyl, 2-Propyloxypropyl, 3-Propyloxy-butyl, Butyloxymethyl oder 2-Butyloxyprop-2-yl;
- $C_3$-$C_8$-Cycloalkyl, bevorzugt $C_3$-$C_6$-Cycloalkyl wie Cyclopropyl, Cyclobutyl, Cyclopentyl und Cyclohexyl;
- $C_4$-$C_{12}$-Cycloalkyl-alkyl, bevorzugt $C_4$-$C_8$-Cycloalkyl-alkyl wie Cyclopropyl-methyl, Cyclopropyl-ethyl, Cyclobutyl-methyl, Cyclopentyl-methyl, Cyclohexyl-methyl und Cyclohexyl-ethyl;
- $C_3$-$C_8$-Halogencycloalkyl, bevorzugt $C_3$-$C_6$-Halogencycloalkyl, besonders bevorzugt $C_3$-$C_6$-Fluor- und Chlorcycloalkyl, wie 2,2-Difluorcyclopropyl, 2,2-Dichlorcyclopropyl;
- $C_4$-$C_{12}$-Halogencycloalkyl-alkyl, bevorzugt $C_4$-$C_8$-Halogencycloalkyl-alkyl, besonders bevorzugt $C_4$-$C_8$-Fluor- und Chlorcycloalkyl-alkyl wie 2,2-Difluorcycloprop-1-yl-methyl, 2,2-Dichlorcycloprop-1-yl-methyl;
- $C_4$-$C_{12}$-Cycloalkyl-halogenalkyl, bevorzugt $C_4$-$C_8$-Cycloalkyl-halogenalkyl, besonders bevorzugt $C_4$-$C_8$-Cycloalkyl-fluor- oder -chloralkyl, wie 2-Cyclopropyl-2-chlorethyl und 2-Cyclopropyl-1,1-difluorethyl;
- $C_4$-$C_{12}$-Halogencycloalkyl-halogenalkyl, bevorzugt $C_4$-$C_8$-Halogencycloalkyl-halogenalkyl, besonders bevorzugt $C_4$-$C_8$-Fluor- und Chlorcycloalkyl-fluor- und -chloralkyl, wie 2,2-Dichlorcycloprop-1-yl-2-chlorethyl.

X
- O, S oder $SO_2$, bevorzugt O und S, besonders bevorzugt O.

Z im Fall von $Q_a$:
- Wasserstoff;
- Halogen, bevorzugt Fluor und Chlor;
- unverzweigtes oder verzweigtes $C_1$-$C_8$-Alkyl, bevorzugt unverzweigtes oder verzweigtes $C_1$-$C_4$-Alkyl, wie Methyl, Ethyl, Propyl, Isopropyl, Butyl, Isobutyl, besonders bevorzugt $C_1$-$C_2$-Alkyl wie Methyl und Ethyl;
- unverzeigtes oder verzweigtes $C_1$-$C_8$-Alkoxy, bevorzugt unverzweigtes oder verzweigtes $C_1$-$C_4$-Alkoxy wie Methoxy, Ethoxy, Propyloxy, Isopropyloxy, Butyloxy, besonders bevorzugt $C_1$-$C_2$-Alkoxy, wie Methoxy und Ethoxy;
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl;
- unverzweigtes oder verzweigtes $C_1$-$C_4$-Halogenalkoxy, bevorzugt $C_1$-$C_2$-Fluor- und Chloralkoxy, besonders bevorzugt Trifluormethoxy und Trichlormethoxy;
- $C_3$-$C_{10}$-Cycloalkyl, bevorzugt $C_3$-$C_6$-Cycloalkyl wie Cyclopropyl, Cyclobutyl, Cyclopentyl und Cyclohexyl, besonders bevorzugt Cyclopropyl;
- Nitro;
- Cyano;

Z im Fall von $Q_b$:
- Wasserstoff, Halogen, bevorzugt Fluor, Chlor und Brom, besonders bevorzugt Fluor und Chlor,
- $C_1$-$C_4$-Alkyl wie Methyl, Ethyl, Propyl, Isopropyl, Butyl,

besonders bevorzugt Methyl und Ethyl.

$R^2$
- bevorzugt Wasserstoff,
- $C_1$-$C_4$-Alkyl wie Methyl, Ethyl, Propyl, Isopropyl oder Butyl, besonders bevorzugt Methyl.

$R^3$-$R^{14}$ unabhängig voneinander
- Wasserstoff;
- Halogen, bevorzugt Fluor und Chlor;
- unverzweigtes oder verzweigtes $C_1$-$C_8$-Alkyl, bevorzugt unverzweigtes oder verzweigtes $C_1$-$C_4$-Alkyl wie Methyl, Ethyl, Propyl, Isopropyl, Butyl, Isobutyl, besonders bevorzugt

$C_1$-$C_2$-Alkyl wie Methyl und Ethyl;

- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl;
- unverzweigtes oder verzeigtes $C_1$-$C_8$-Alkoxy, bevorzugt unverzweigtes oder verzweigtes $C_1$-$C_4$-Alkoxy wie Methoxy, Ethoxy, Propyloxy, Isopropyloxy, Butyloxy, besonders bevorzugt $C_1$-$C_2$-Alkoxy wie Methoxy und Ethoxy;
- $C_3$-$C_{10}$-Cycloalkyl, bevorzugt $C_3$-$C_6$-Cycloalkyl wie Cyclopropyl, Cyclobutyl, Cyclopentyl und Cyclohexyl, besonders bevorzugt Cyclopropyl;
- Aryl; bevorzugt Phenyl, 1-Naphthyl und 2-Naphthyl, besonders bevorzugt Phenyl;
- ein- bis dreifach durch Halogen substituiertes Aryl, bevorzugt durch Fluor oder Chlor monosubstituiertes Phenyl wie 2-Fluorphenyl, 3-Fluorphenyl, 4-Fluorphenyl, 2-Chlorphenyl, 3-Chlorphenyl und 4-Chlorphenyl;
- ein- bis dreifach durch unverzweigtes oder verzweigtes $C_1$-$C_8$-Alkyl substituiertes Aryl, bevorzugt durch unverzweigtes oder verzweigtes $C_1$-$C_4$-Alkyl monosubstituiertes Phenyl, besonders bevorzugt durch $C_1$-$C_2$-Alkyl monosubstituiertes Phenyl wie 4-Methylphenyl und 4-Ethylphenyl;
- ein- bis dreifach durch unverzweigtes oder verzweigtes $C_1$-$C_8$-Alkoxy substituiertes Phenyl, besonders bevorzugt durch $C_1$-$C_2$-Alkoxy monosubstituiertes Phenyl, wie 4-Methoxyphenyl und 4-Ethoxyphenyl;
- ein- bis dreifach durch unverzweigtes oder verzweigtes $C_1$-$C_4$-Halogenalkyl substituiertes Aryl, bevorzugt durch $C_1$-$C_2$-Fluor- und Chloralkyl monosusbtituiertes Phenyl, besonders bevorzugt durch Trifluormethyl und Trichlormethyl monosubstituiertes Phenyl, wie 4-Trifluormethylphenyl und 4-Trichlormethylphenyl;
- ein- bis dreifach durch unverzweigtes oder verzweigtes $C_1$-$C_4$-Halogenalkoxy substituiertes Aryl, bevorzugt durch $C_1$-$C_2$-Fluor- und Chloralkoxy monosubstituiertes Phenyl, besonders bevorzugt durch Trifluormethoxy und Trichlormethoxy monosubstituiertes Phenyl wie Trifluormethoxyphenyl und 4-Trichlormethoxyphenyl;

$Q_b$

- ein gegebenenfalls substituierter Fünfringheteroaromat mit 1 bis 4, insbesondere 1 und 2 Heteroatomen wie Stickstoff, Schwefel oder Sauerstoff, bevorzugt Thiophen-2-yl, Thiophen-3-yl, Thiazol-5-yl, Thiazol-4-yl, Thiazol-2-yl, Oxazol-5-yl, Oxazol-4-yl, Oxazol-2-yl, Imidazol-5-yl, Imidazol-4-yl, Imidazol-2-yl, Isothiazol-5-yl, Isothiazol-3-yl, Pyrazol-3-yl, Pyrazol-4-yl, Pyrazol-5-yl, Isoxazol-5-yl, Isoxazol-3-yl, 1,3,4-Thiadiazol-2-yl, 1,3,4-Oxadiazol-2-yl, 1,2,4-Oxadiazol-3-yl, 1,3,4-Triazol-2-yl, 1,2,4-Thiadiazol-3-yl, besonders bevorzugt Thiophen-2-yl, Thiophen-3-yl, Thiazol-4-yl, Imidazol-5-yl, Pyrazol-3-yl, 1,3,4-Thiadiazol-2-yl, 1,3,4-Oxadiazol-2-yl, 1,2,4-Oxadiazol-3-yl, Isoxazol-5-yl und Isoxazol-3-yl.

Der Fünfringhetarylrest kann gegebenenfalls ein- oder mehrfach substituiert sein mit:

- Halogen, bevorzugt Fluor, Chlor und Brom, besonders bevorzugt Chlor und Brom,
- $C_1$-$C_8$-Alkyl, bevorzugt $C_1$-$C_4$-Alkyl, besonders bevorzugt Methyl, Ethyl, Isopropyl, tert.-Butyl,
- $C_2$-$C_8$-Alkenyl, bevorzugt $C_2$-$C_4$-Alkenyl, besonders bevorzugt Ethenyl, 1-Methyl-ethenyl, Propenyl, 2-Methyl-propenyl,
- $C_1$-$C_4$-Halogenalkyl, bevorzugt mit Fluor oder Chlor substituiertes $C_1$-$C_2$-Halogenalkyl, besonders bevorzugt Trifluormethyl, 2,2,2-Trifluoreth-1-yl,
- $C_1$-$C_8$-Alkoxy, bevorzugt $C_1$-$C_3$-Alkoxy, besonders bevorzugt Methoxy, Ethoxy, n-Propyloxy und Isopropyloxy,
- $C_1$-$C_8$-Alkylthio, bevorzugt $C_1$-$C_3$-Alkylthio, besonders bevorzugt Methylthio, Ethylthio, n-Propylthio und Isopropylthio,
- $C_2$-$C_8$-Alkoxyalkyl, bevorzugt $C_2$-$C_4$-Alkoxyalkyl, besonders bevorzugt Methoxymethyl, 1-Methoxyethyl, 2-Methoxyethyl, 1-Methoxy-propyl,
- $C_3$-$C_8$-Cycloalkyl, bevorzugt $C_3$-$C_5$-Cycloalkyl wie Cyclopropyl, Cyclobutyl und Cyclopentyl.

Die erfindungsgemäßen Verbindungen Ia bzw. Ib können ein oder mehrere Asymmetriezentren im Substituenten $R^1$ enthalten. Die vorliegende Erfindung schließt alle möglichen Stereoisomeren wie Diastereomeren, Enantiomeren, Diastereomeren- und Enantiomerengemische ein. Bei den in der Tabelle der Beispielverbindungen betreffenden Verbindungen handelt es sich um das jeweilige racemische Gemisch.

Die phenoxyalkylsubstituierten Heteroaromaten der allgemeinen Formeln Ia und Ib sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämp-

fungsmitteleingesetzt werden.

Sie können daher in Verfahren zur Bekämpfung von Schädlingen eingesetzt werden, wobei man eine für Schädlinge wirksame Menge eines phenoxyalkylsubstituierten 5-Ring-Heteroaromaten der Formel Ia oder Ib auf Schädlinge bzw. deren Lebensraum einwirken läßt.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinnstmotte), Argyresthia conjugella (Apfelmotte), Sitotroga corealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsischtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalls (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (weißer Bärenspinner), Agrotis segetum (Wintersaateule), Agrotis ypsilon (Ypsiloneule),Barathra brassicae (Kohleule), Cirphis unipuncta (Heuwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pierus brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer) Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Meolontha melolontha (Feldmaikäfer), Melolontha hippocastani (Waldmaikäfer), Amphimallus solstitialis (Brachkäfer), Crioceris aspargi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus salcatus (Gefurchter Lappenrüßler), Ortiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler) Sitophilus granaria (Kornkäfer), Anisandrus dispar (Ungleicher Holzborkenkäfer), Ips typographus (Buchdrucker), Bastophagus piniperda (Gefruchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke) Tipula plaudose (Wiesenschnacke), Tipula oleracea (Kohlschnacke), Dacus cucurbitae (Melonenfliege), Dacusoleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludose (Wiesenschnacke), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rose (Rübenblattwespe), Hoplocampa minuta (Pflaumensägewespe), Monomorium pharaonis (Pharaoameise), solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus),

Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dyasphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tanntrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausheuschrecke), Lousta migratoria (Wanderheuschrecke), Stauronotus maroccanus (Marrokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Kücheschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplusm Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, heterodera avenae, Heterodera glycinae, Heterodera triflolii, Stock- und Blattälchen, z.B. Ditylenchus dipsaci, Ditylenchus destructor, Pratylenchus neglectus, Pratylenchus penetrans, Paratylenchus goodeyi, Paratylenchus curvitatus sowie Tylenchorrhynchus dubius, Tylenchorhynchus claytoni, Rotylenchus robustus, Heliocotylenchus multicinctus, Radopholus similis, Belonolaimus longicaudatus, Longidorus elongatus, Trichodrus primitivus.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten, netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel in Wasser homogenisiert werden.

Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkai- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate z.B. Umhüllungs- und Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Beispiele für Formulierungen sind:

I. 5 Gew.-Teile der Verbindung Nr. A1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. A4 werden mit einer Mischung au 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. A50 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecyl benzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. B1 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. B10 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 Gew.%. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 10, insbesondere 0,1 bis 2, vorzugsweise 0,01 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele

A) N-Phenoxymethylsubstituierte Azole ($Q_a$)

1-[4-(1-Methyl-1-proploxy)-phenoxymethyl]-4,5-dichlorimidazol (Verbindung Nr. A4)

Zu 0,79 g 80 %igem Natriumhydrid in 50 ml trockenem Dimethylformamid tropft man unter Stickstoffatmosphäre bei Raumtemperatur (ca. 20 °C) 5,0 g 4-(1-Methyl-1-propyloxy)-phenol in 20 ml trockenem Dimethylformamid. Nach dem Abklingen der exothermen Reaktion (Wasserstoffentwicklung) läßt man noch 30 Minuten bei 60 °C nachrühren und tropft anschließend bei Raumtemperatur (ca. 20 °C) 6,12 g 1-Chlormethyl-4,5-dichlorimidazol in 20 ml trockenem Dimethylformamid zu. Anschließend rührt man 6 Stunden bei 80 °C und über Nacht bei Raumtemperatur (ca. 20 °C) nach. Zur Aufarbeitung gießt man in 150 ml Eiswasser und extrahiert dreimal mit Essigester. Die organischen Phasen werden mit 5 %iger Natronlauge und mit Wasser gewaschen. Nach dem Trocknen über Natriumsulfat und dem Einengen i. Vak. wird das

Rohprodukt durch Chromatographie an Kieselgel mit Toluol/Essigester = 9/1 als Eluent gereinigt. Man erhält 4,3 g 1-[4(1-Methyl-1-propyloxy)-phenoxymethyl]-4,5-dichlorimidazol mit $n_D^{22}$ = 1,5475.

1-[4-(3,3-Dimethyl-1-propyloxy)-phenoxymethyl]-4,5-dichlorimidazol (Verbindung Nr. A50)

Zu 0,79 g 80 %igem Natriumhydrid in 50 ml trockenem Dimethylformamid tropft man unter Stickstoffatmosphäre bei Raumtemperatur 5,8 g 4-(3,3-Dimethyl-1-propyloxy)-phenol in 20 ml trockenem Dimethylformamid. Nach dem Abklingen der Wasserstoffentwicklung läßt man noch 1 Stunde bei 60 °C nachrühren und tropft anschließend bei Raumtemperatur (ca. 20 °C) 6,12 g 1-Chlormethyl-4,5-dichlorimidazol in 20 ml trockenem Dimethylformamid zu. Anschließend rührt man 3 Stunden bei 80 °C und über Nacht bei Raumtemperatur (ca. 20 °C) nach. Zur Aufarbeitung gießt man auf 100 ml Eiswasser und extrahiert dreimal mit Methyl-tert.-butylether. Die organischen Phasen werden anschließend mit 5 %iger Natronlauge und dreimal mit Wasser gewaschen, über Natriumsulfat getrocknet und i. Vak. eingeengt. Der Rückstand wird aus Cyclohexan umkristallisiert. Man erhält 7,4 g 1-[4-(3,3-Dimethyl-1-propyloxy)-phenoxymethyl]-4,5-dichlorimidazol mit Fp.: 105-108 °C.

Entsprechend dieser Herstellvorschriften können die in den folgenden Tabellen 1 bis 6 beschriebenen Verbindungen Iaa bis Iaf hergestellt werden.

Die ohne Angabe physikalischer Kenngrößen in den nachstehenden Tabellen 1 bis 6 aufgeführten Verbindungen Iaa bis Iaf können aus entsprechenden Vorprodukten ohne weiteres erhalten werden und lassen eine gleichartige Wirkung erwarten.

Tabelle 1

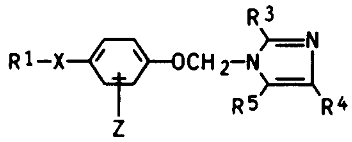

(Iaa)

| Nr. | $R^1$ | Z | $R^3$ | $R^4=R^5$ | X | phys. Daten |
|---|---|---|---|---|---|---|
| A1 | $(CH_3)_2CH-$ | H | H | Cl | O | $n_D^{24}$:1.5421 |
| A2 | $(CH_3)_2CH-$ | H | H | Cl | S | |
| A3 | $(CH_3)_2CH-$ | H | H | Cl | $SO_2$ | |
| A4 | $CH_3CH_2CH(CH_3)-$ | H | H | Cl | O | $n_D^{23}$:1.5475 |
| A5 | $CH_3CH_2CH(CH_3)-$ | H | H | Cl | S | $n_D^{24}$:1.5795 |
| A6 | $CH_2CH_2CH(CH_3)-$ | H | H | Cl | $SO_2$ | Fp.: 111-114°C |
| A7 | $CH_3CH_2CH(CH_3)-$ | 3-F | H | Cl | O | |
| A8 | $CH_3CH_2CH(CH_3)-$ | 3-F | H | Cl | S | |
| A9 | $CH_3CH_2CH(CH_3)-$ | 3-F | H | Cl | $SO_2$ | |
| A10 | $CH_3CH_2CH(CH_3)-$ | 3-Cl | H | Cl | O | |
| A11 | $CH_3CH_2CH(CH_3)-$ | 3-Cl | H | Cl | S | |
| A12 | $CH_3(CH_2)_2CH(CH_3)-$ | H | H | Cl | O | |
| A13 | $CH_3(CH_2)_2CH(CH_3)-$ | H | H | Cl | S | |
| A14 | $CH_3(CH_2)_3CH(CH_3)-$ | H | H | Cl | O | |
| A15 | $CH_3(CH_2)_3CH(CH_3)-$ | H | H | Cl | S | |
| A16 | $CH_3(CH_2)_4CH(CH_3)-$ | H | H | Cl | O | |

| Nr. | $R^1$ | Z | $R^3$ | $R^4=R^5$ | X | phys. Daten |
|---|---|---|---|---|---|---|
| A17 | 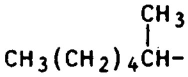 | H | H | Cl | S | |
| A18 | 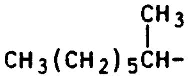 | H | H | Cl | O | |
| A19 | 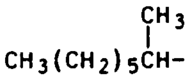 | H | H | Cl | S | |
| A20 | 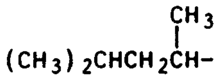 | H | H | Cl | O | $n_D^{23}$:1.5373 |
| A21 | 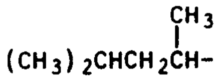 | H | H | Cl | S | |
| A22 | 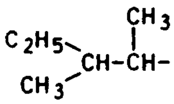 | H | H | Cl | O | |
| A23 | 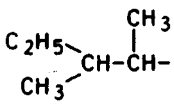 | H | H | Cl | S | |
| A24 | 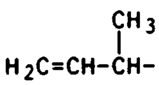 | H | H | Cl | O | |
| A25 | 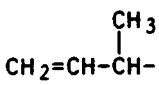 | H | H | Cl | S | |
| A26 | 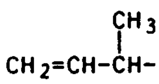 | H | H | Cl | $SO_2$ | |
| A27 | $CH_2=CH-CH_2-$ | H | H | Cl | O | |
| A28 | $CH_2=CH-CH_2-$ | H | H | Cl | S | |
| A29 | 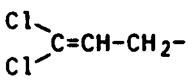 | H | H | Cl | O | |
| A30 | 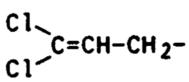 | H | H | Cl | S | |
| A31 | 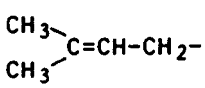 | H | H | Cl | O | |
| A32 | 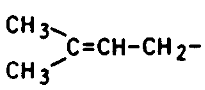 | H | H | Cl | S | |
| A33 | Cylcobutyl | H | H | Cl | O | |
| A34 | Cyclobutyl | H | H | Cl | S | |
| A34 | Cyclopentyl | H | H | Cl | O | |

| Nr. | $R^1$ | Z | $R^3$ | $R^4$=$R^5$ | X | phys. Daten |
|---|---|---|---|---|---|---|
| A35 | Cyclopentyl | H | H | Cl | S | |
| A36 | Cyclohexyl | H | H | Cl | O | |
| A37 | Cyclohexyl | H | H | Cl | S | |
| A38 | Cyclopropyl-methyl | H | H | Cl | O | |
| A39 | Cyclohexyl-methyl | H | H | Cl | O | |
| A40 | 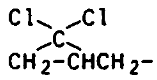 | H | H | Cl | O | Fp.: 94-96°C |
| A41 | 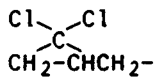 | H | H | Cl | S | |
| A42 | 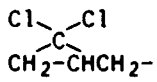 | H | H | Cl | $SO_2$ | |
| A43 | 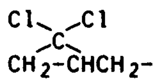 | 3-F | H | Cl | O | |
| A44 | $HC{\equiv}C-CH_2-$ | H | H | Cl | O | |
| A45 | $ClC{\equiv}C-CH_2-$ | H | H | Cl | O | |
| A46 | 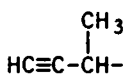 | H | H | Cl | O | |
| A47 | 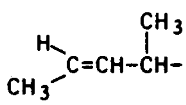 | H | H | Cl | O | |
| A48 | $(CH_3)_2CHCH_2-$ | H | H | Cl | O | Fp.: 65-68°C |
| A49 | $(CH_3)_2CH(CH_2)_2-$ | H | H | Cl | O | |
| A50 | $(CH_3)_2CHCH_2-$ | H | H | Cl | S | |
| A51 | $(C_2H_5)_2CH-$ | H | H | Cl | O | $n_D^{22}$: 1.5475 |
| A52 | $(CH_2F)_2CH-$ | H | H | Cl | O | |
| A53 | $CF_3CH_2-$ | H | H | Cl | O | |
| A54 | 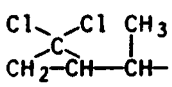 | H | H | Cl | O | |
| A55 | $ClCH=CHCH_2-$ | H | H | Cl | O | |
| A56 | $(CH_3)_3CCH_2-$ | H | H | Cl | O | Fp.: 105-108°C |
| A57 | $CH_3CH_2CH_2-$ | H | H | Cl | O | |
| A58 | $CH_3CH_2CH_2CH_2-$ | H | H | Cl | O | |
| A59 | $CH_3-$ | H | H | Cl | O | |
| A60 | $CH_3CH_2-$ | H | H | Cl | O | |
| A61 | $CH_3(CH_2)_2CH_2-$ | H | H | Cl | S | |

| Nr. | $R^1$ | Z | $R^3$ | $R^4=R^5$ | X | phys. Daten |
|---|---|---|---|---|---|---|
| A62 | $(CH_3)_3C(CH_2)_2-$ | H | H | Cl | O | |
| A63 | $CH_3$<br>$(CH_3)_3CCH_2CH-$ | H | H | Cl | O | |
| A64 | $CH_3$<br>$CH_3CH_2CH-CH_2-$ | H | H | Cl | O | |
| A65 | $C_2H_5$<br>$CH_3(CH_2)_2-CH-$ | H | H | Cl | O | $n_D^{22}$: 1.5365 |
| A66 | $CH_3$<br>$(CH_3)_2CH-CH-$ | H | H | Cl | O | $n_D^{22}$: 1.5421 |
| A67 | $CH_3$<br>$(CH_3)_2CH-CH-$ | H | H | Cl | O | |
| A68 | $CH_3$<br>$C=CH-CH_2-$<br>$CH_3$ | H | H | Cl | O | |
| A69 | $CH_3$<br>$CH_3CHCH-$<br>F | H | H | Cl | O | |
| A70 | $CH_3$<br>$ClCH_2CH-$ | H | H | Cl | O | |
| A71 | $CH_3$<br>$Cl(CH_2)_2CH-$ | H | H | Cl | O | |
| A72 | $CH_3$<br>$CH_3OCHCH_2-$ | H | H | Cl | O | |
| A73 | $CH_3$<br>$CH_3OCH-(CH_2)_2-$ | H | H | Cl | O | |
| A74 | $CH_3$<br>$CH_3OCH_2-CH$ | H | H | Cl | O | $n_D^{22}$: 1.5500 |
| A75 | $CH_3CH_2O(CH_2)_2-$ | H | H | Cl | O | $n_D^{22}$: 1.5500 |
| A76 | $CH_3$<br>$CH_3O(CH_2)_2CH-$ | H | H | Cl | O | |
| A77 | $CH_3O(CH_2)_2-$ | H | H | Cl | O | $n_D^{22}$: 1.5575 |
| A78 | $CH_3$<br>$CH_3OCH_2CH-$ | H | H | Cl | S | |
| A79 | $CH_3$<br>$CH_3CH_2CH-$ | H | H | H | O | |

| Nr. | $R^1$ | Z | $R^3$ | $R^4=R^5$ | X | phys. Daten |
|---|---|---|---|---|---|---|
| A80 | $CH_3$<br>\|<br>$CH_3CH_2CH-$ | H | H | H | S | |
| A81 | $CH_3$<br>\|<br>$CH_3CH_2CH-$ | H | H | H | $SO_2$ | |
| A82 | $(CH_3)_2CH-$ | H | H | H | O | |
| AA3 | 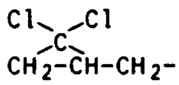 | H | H | H | O | |
| A84 | $CH_3O(CH_2)_2-$ | H | H | H | O | |
| A85 | $CH_3$<br>\|<br>$CH_3OCH_2CH-$ | H | H | H | O | |
| A86 | $CH_3$<br>\|<br>$(CH_3)_2CHCH_2CH-$ | H | H | H | O | |
| A87 | $CH_3$<br>\|<br>$(CH_3)_2CHCH_2CH-$ | H | H | $CH_3$ | O | |
| A88 | $CH_3$<br>\|<br>$CH_3OCH_2CH-$ | H | H | $CH_3$ | O | |
| A89 | $CH_3O(CH_2)_2-$ | H | H | $CH_3$ | O | |
| A90 | $(CH_3)_2CH-$ | H | H | $CH_3$ | O | |
| A91 | 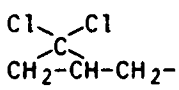 | H | H | $CH_3$ | O | |
| A92 | $CH_3$<br>\|<br>$CH_3CH_2CH-$ | H | H | $CH_3$ | O | |
| A93 | $CH_3$<br>\|<br>$CH_3CH_2CH-$ | H | H | $CH_3$ | S | |
| A94 | $CH_3$<br>\|<br>$CH_3CH_2CH-$ | H | H | $CH_3$ | $SO_2$ | |

Tabelle 2

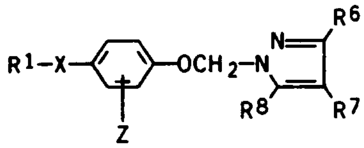 (Iab)

| Nr. | $R^1$ | Z | $R^6$ | $R^7$ | $R^8$ | X | phys. Daten |
|---|---|---|---|---|---|---|---|
| A95 | $(CH_3)_2CH-$ | H | H | H | H | O | |
| A96 | $(CH_3)_2CH-$ | H | H | H | H | S | |
| A97 | 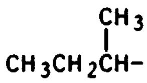 | H | H | H | H | O | |
| A98 | 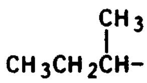 | H | H | H | H | S | |
| A99 | 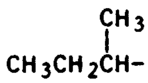 | H | H | H | H | $SO_2$ | |
| A100 | $CH_3OCH_2CH_2-$ | H | H | H | H | O | |
| A101 | $CH_3OCH_2CH_2-$ | H | H | H | H | S | |
| A102 | 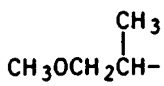 | H | H | H | H | O | |
| A103 | 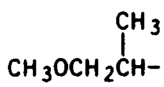 | H | H | H | H | S | |
| A104 | 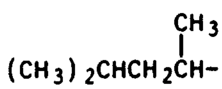 | H | H | H | H | O | |
| A105 | 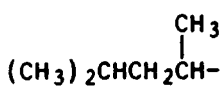 | H | H | H | H | S | |
| A106 | 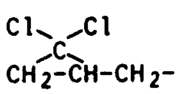 | H | H | H | H | O | |
| A107 | 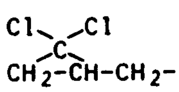 | H | H | H | H | S | |
| A108 | 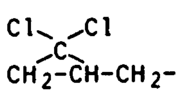 | H | H | H | H | $SO_2$ | |

Tabelle 3

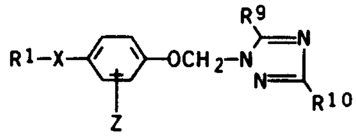

(Iac)

| Nr. | $R^1$ | Z | $R^9=R^{10}$ | X | phys. Daten |
|---|---|---|---|---|---|
| A109 | 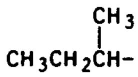 | H | H | O | $n_D^{21}$: 1.5310 |
| A110 | 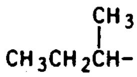 | H | H | S | $n_D^{21}$: 1.5665 |
| A111 | 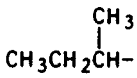 | H | H | $SO_2$ | |
| A112 | 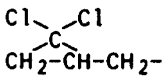 | H | H | O | $n_D^{21}$: 1.5635 |
| A113 | 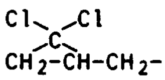 | H | H | S | |
| A114 | $(CH_3)_2CH-$ | H | H | O | $n_D^{21}$: 1.5360 |
| A115 | $(CH_3)_2CH-$ | H | H | S | |
| A116 | $CH_3O(CH_2)_2-$ | H | H | O | |
| A117 | 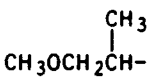 | H | H | O | |
| A118 | 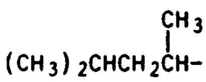 | H | H | O | $n_D^{21}$: 1.5210 |
| A119 | 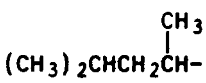 | H | $CH_3$ | O | |
| A120 | 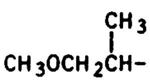 | H | $CH_3$ | O | |
| A121 | $CH_3O(CH_2)_2-$ | H | $CH_3$ | O | |
| A122 | $(CH_3)_2CH-$ | H | $CH_3$ | O | |
| A123 | 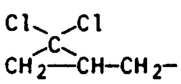 | H | $CH_3$ | O | |
| A124 | 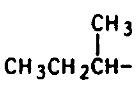 | H | $CH_3$ | $SO_2$ | |
| A125 | 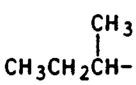 | H | $CH_3$ | S | |
| A126 | 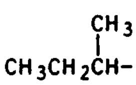 | H | $CH_3$ | O | |

Tabelle 4

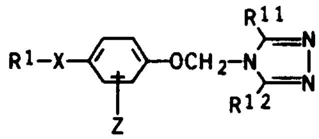

(Iad)

| Nr. | $R^1$ | Z | $R^{11}$ | $R^{12}$ | X | phys. Daten |
|---|---|---|---|---|---|---|
| A127 | 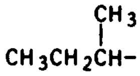 | H | H | Cl | O | |
| A128 | 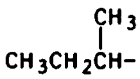 | H | H | Cl | S | |
| A129 | 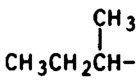 | H | H | Cl | $SO_2$ | |
| A130 | 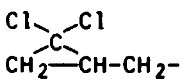 | H | H | Cl | O | |
| A131 | 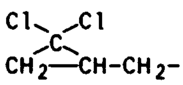 | H | H | Cl | S | |
| A132 | $(CH_3)_2CH-$ | H | H | Cl | O | |
| A133 | $CH_3O(CH_2)_2-$ | H | H | Cl | O | |
| A134 | 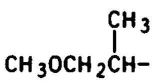 | H | H | Cl | O | |
| A135 | 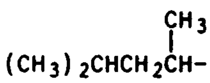 | H | H | Cl | O | |
| A136 | 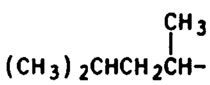 | H | H | H | O | |
| A137 | 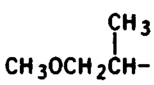 | H | H | H | O | |
| A138 | $CH_3O(CH_2)_2-$ | H | H | H | O | |
| A139 | $(CH_3)_2CH-$ | H | H | H | O | |
| A140 | 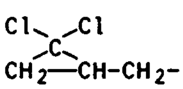 | H | H | H | O | |
| A141 | 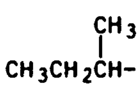 | H | H | H | $SO_2$ | |
| A142 | 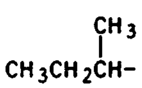 | H | H | H | S | |
| A143 | 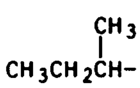 | H | H | H | O | |

Tabelle 5

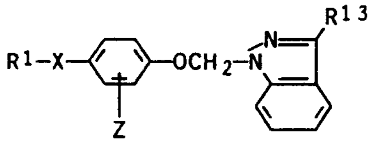

(Iae)

| Nr. | $R^1$ | Z | $R^{13}$ | X | phys. Daten |
|---|---|---|---|---|---|
| A144 | $CH_3$<br>$CH_3CH_2CH-$ | H | H | O | $n_D^{25}$: 1.5755 |
| A145 | $CH_3$<br>$CH_3CH_2CH-$ | H | H | S | |
| A146 | $CH_3$<br>$CH_3CH_2CH-$ | H | H | $SO_2$ | |
| A147 | Cl Cl<br>C<br>$CH_2$—CH—$CH_2$– | H | H | O | |
| A148 | $(CH_3)_2CH-$ | H | H | O | $n_D^{25}$: 1.5817 |
| A149 | $CH_3O(CH_2)_2-$ | H | H | O | |
| A150 | $CH_3$<br>$CH_3OCH_2CH-$ | H | H | O | $n_D^{25}$: 1.5745 |
| A151 | $CH_3$<br>$(CH_3)_2CH_2CH-$ | H | H | O | |

Tabelle 6

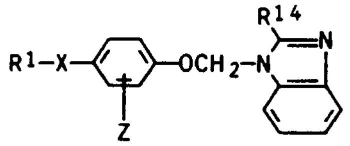

(Iaf)

| Nr. | $R^1$ | Z | $R^3$ | $R^{14}$ | X | phys. Daten |
|---|---|---|---|---|---|---|
| A152 | $CH_3$<br>$CH_3CH_2CH-$ | H | H | H | O | |
| A153 | $CH_3$<br>$CH_3CH_2CH-$ | H | H | H | S | |
| A154 | $CH_3$<br>$CH_3CH_2CH-$ | H | H | H | $SO_2$ | |
| A155 | 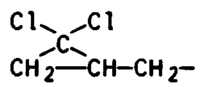 | H | H | H | O | |
| A156 | $(CH_3)_2CH-$ | H | H | H | O | |
| A157 | $CH_3O(CH_2)_2-$ | H | H | H | O | |
| A158 | $CH_3$<br>$CH_3OCH_2CH-$ | H | H | H | O | |
| A159 | $CH_3$<br>$(CH_3)_2CHCH_2CH-$ | H | H | H | O | |
| A160 | $CH_3$<br>$(CH_3)_2CHCH_2CH-$ | H | H | $CH_3$ | O | |

B) Phenoxyalkylsubstituierte Fünfringheteroaromaten ($Q_b$)

5-[4-(sec-Butoxy)-phenoxymethyl]-3-methyl-isoxazol (Bsp.-Nr. B1 der nachfolgenden Tabelle):

5,0 g 4-sec-Butyloxyphenol werden in 80 ml absolutem Dimethylformamid vorgelegt und mit 4,6 g wasserfreiem Kaliumcarbonat 1 Stunde bei 80 °C gerührt. Anschließend tropft man bei dieser Temperatur 3,9 g 5-Chlormethyl-3-methyl-isoxazol zu und läßt noch 7 Stunden bei 90 °C nachrühren. Zur Aufarbeitung wird der Ansatz in 300 ml Eiswasser eingerührt, dreimal mit je 100 ml Methyl-tert.-butylether extrahiert und die organische Phase zweimal mit je 50 ml 2 N Natronlauge und anschließend dreimal mit je 50 ml Wasser gewaschen. Nach Trocknen über Natriumsulfat und Abziehen des Lösungsmittels im Vakuum erhält man 6,6 g 5-[4-(sec-Butyloxy)-phenoxymethyl]-3-methyl-isoxazol, welches nach Chromatographie über Kieselgel mit Toluol/Essigester = 1/1 $^1$H-NMR-spektroskopisch rein als viskoses Öl anfällt.
250-MHz-$^1$H-NMR in $CDCl_3$ [ppm], (charakteristische Signale): 1.21 (d); 5.08 (s); 6.15 (s).

5-[4-(sec-Butylthio)-phenoxymethyl]-3-cyclopropyl-isoxazol (Bsp.-Nr. B10 der nachfolgenden Tabelle):

0,79 g Natriumhydrid werden unter Stickstoff in 50 ml absolutem Dimethylformamid vorgelegt. Hierzu tropft man bei Raumtemperatur 5,5 g 4-sec-Butyl-thiophenol in 20 ml absolutem Dimethylformamid gelöst zu und rührt anschließend noch 0,5 Stunden bei 60 °C nach. Anschließend werden bei dieser Temperatur 5,8 g 5-Chlormethyl-3-cyclopropyl-isoxazol in 20 ml absolutem Dimethylformamid zugetropft und nach beendeter Zugabe noch 3 Stunden bei 90 °C nachgerührt. Analoge Aufarbeitung wie im vorangegangenen Beispiel liefert nach Chromatographie über Kieselgel mit Toluol/Essigester = 1/1 7,9 g 5-[4-(sec-Butylthio)-phenoxymethyl]-3-cyclopropyl-isoxazol als hellgelbes Öl.
250-MHz-$^1$H-NMR in $CDCl_3$ [ppm], (charakteristische Signale): 1.22 (d); 5.09 (s); 6.01 (s).

2-[4-(sec-Butyloxy)-phenoxymethyl]-5-bromthiophen (Bsp.-Nr. B49 der nachfolgenden Tabelle):

6,64 g 4-(sec-Butyloxy)-phenol und 5,52 g Kaliumcarbonat werden in 50 ml trockenem Dimethylformamid 1 Stunde auf 70°C erhitzt. Anschließend tropft man 8,46 g 5-Brom-2-chlormethylthiophen in 20 ml trockenem Dimethylformamid zu. Man rührt 6 Stunden bei 80°C und über Nacht bei Raumtemperatur (ca. 20°C) nach. Zur Aufarbeitung gießt man in 100 ml Eiswasser und extrahiert dreimal mit Essigester und trocknet die organischen Phasen über Magnesiumsulfat. Nach dem Verdampfen des Lösungsmittels wird das Rohprodukt durch Chromatographie an Kieselgel mit n-Hexan/Essigester = 4/1 als Eluent gereinigt. Man erhält 6,3 g 2-[4-(sec-Butyloxy)-phenoxymethyl]-5-brom-thiophen als farbloses Öl.
250-MHz-$^1$H-NMR in $CDCl_3$ [ppm], (charakteristische Signale): 0.99 (t); 1.28 (d); 5.09 (s).

5-[4-(sec-Butyloxy)-phenoxymethyl]-3-cyclopropyl-1,2,4-oxadiazol (Bsp.-Nr. B69 der nachfolgenden Tabelle):

5 g 4-(sec-Butyloxy)-phenol und 6,2 g Kaliumcarbonat werden in 50 ml trockenem Dimethylformamid 1 Stunde bei 80°C gerührt. Anschließend werden 4,8 g 5-Chlormethyl-3-cyclopropyl-1,2,4-oxadiazol in 20 ml trockenem Dimethylformamid zugetropft und 8 Stunden bei 80°C und über Nacht bei Raumtemperatur (ca. 20°C) nachgerührt. Zur Aufarbeitung gießt man in 100 ml Wasser und extrahiert dreimal mit Essigester und trocknet die organischen Phasen über Magnesiumsulfat. Nach dem Verdampfen des Lösungsmittels wird das Rohprodukt durch Chromatographie am Kieselgel mit n-Hexan/Essigester = 4/1 als Eluent gereinigt. Man erhält 6,7 g 5-[4-(sec-Butyloxy)-phenoxymethyl]-3-cyclopropyl-1,2,4-oxadiazol als farbloses Öl.
250 MHz-$^1$H-NMR in $CDCl_3$ [ppm], (charakteristische Signale): 1.50-1.82 (m); 2.04-2.18 (m); 5.13 (s).

Tabelle 7

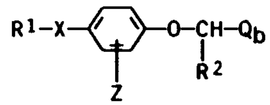

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B1 | $CH_3CH_2\overset{CH_3}{\overset{\vert}{C}}H-$ | H | H | O | 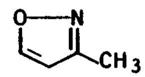 | 5 | 1.21 (d), 5.08 (s), 6.15 (s) |
| B2 | $(CH_3)_2CHCH_2CH_2-$ | H | H | O | 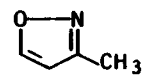 | 5 | 0.98 (d), 2.31 (s): 6.18 (s) |
| B3 | $CH_3CH_2CH_2\overset{CH_3}{\overset{\vert}{C}}H-$ | H | H | O | 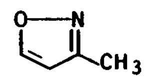 | 5 | 0.92 (t), 1.26 (d), 5.09 (s) |
| B4 | $(CH_3)_2CHCH_2\overset{CH_3}{\overset{\vert}{C}}H-$ | H | H | O | 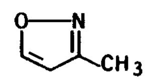 | 5 | 0.93 (t), 1.22 (d), 2.31 (s) |
| B5 | $CH_3(CH_2)_3\overset{CH_3}{\overset{\vert}{C}}H-$ | H | H | O | 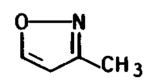 | 5 | 2.28 (s), 5.08 (s), 6.17 (s) |
| B6 | $CH_3CH_2\overset{CH_3}{\overset{\vert}{C}}H-$ | H | H | S | 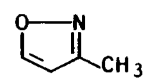 | 5 | 2.30 (s), 5.12 (s), 6.19 (s) |
| B7 | $(CH_3)_2CH-$ | H | H | O | 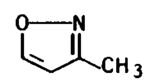 | 5 | 1.31 (d), 2.33 (s), 6.19 (s) |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B8 | $(CH_3)_3CCH_2CH_2-$ | H | H | O | 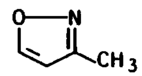 | 5 | 0.98 (s), 2.31 (s), 5.10 (s) |
| B9 | 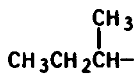 | H | H | O | 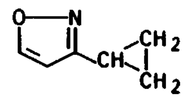 | 5 | 1.22 (d), 5.10 (s), 5.98 (s) |
| B10 | 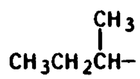 | H | H | S | 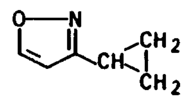 | 5 | 1.22 (d), 5.09 (s), 6.01 (s) |
| B11 | 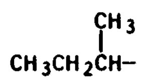 | H | H | $SO_2$ | 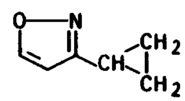 | 5 | 1.05 (t), 5.22 (s), 6.09 (s) |
| B12 | 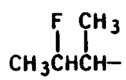 | H | H | O | 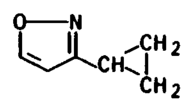 | 5 | 1.32 (d), 4.21-4.83 (m), 5.02 (s) |
| B13 | $(CH_3)_2CH-$ | H | H | O | 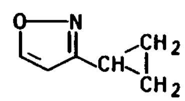 | 5 | 1.35 (d), 5.08 (s), 6.89 (s) |
| B14 | 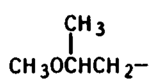 | H | H | O | 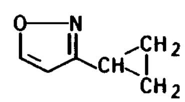 | 5 | 1.28 (d), 3.46 (s), 5.98 (s) |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B15 | $CH_3OCH_2CH_2-$ | H | H | O | 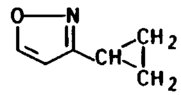 | 5 | 3.42 (s), 3.63-3.71 (m), 3.91-4.05 (m) |
| B16 | 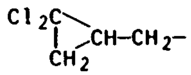 | H | H | O | 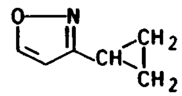 | 5 | 4.08 (d), 5.05 (s), 6.00 (s) |
| B17 | 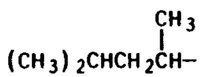 | H | H | O | 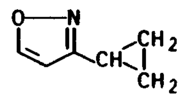 | 5 | 1.23 (d), 4.98 (s), 5.96 (s) |
| B18 | 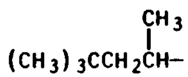 | H | H | O | 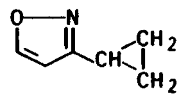 | 5 | |
| B19 | 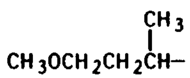 | H | H | O | 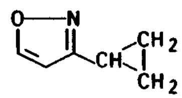 | 5 | 3.48 (s), 5.07 (s), 6.00 (s) |
| B20 | 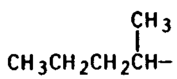 | H | H | O | 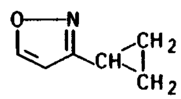 | 5 | |
| B21 | 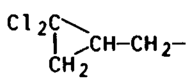 | H | H | S | 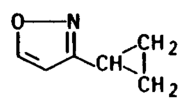 | 5 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B22 | $CH_3OCH_2CH_2-$ | H | H | S | 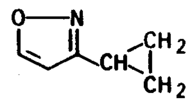 | 5 | |
| B23 | $CH_3OCH_2\overset{CH_3}{\overset{\vert}{C}}H-$ | H | H | S | 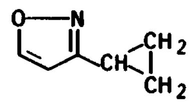 | 5 | |
| B24 | $C_2H_5OCH_2CH_2-$ | H | H | O | 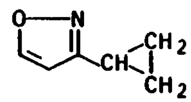 | 5 | |
| B25 | $CH_3CH_2\overset{CH_3}{\overset{\vert}{C}}H-$ | H | H | O | 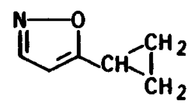 | 3 | |
| B26 | $CH_3CH_2\overset{CH_3}{\overset{\vert}{C}}H-$ | H | H | S | 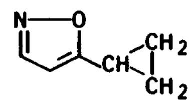 | 3 | |
| B27 | $CH_3CH_2\overset{CH_3}{\overset{\vert}{C}}H-$ | H | H | $SO_2$ | 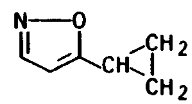 | 3 | |
| B28 | $(CH_3)_2CH-$ | H | H | O | 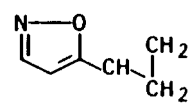 | 3 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| A29 | 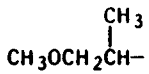 | H | H | O | 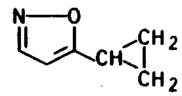 | 3 | 1.27 (d), 3.40 (s), 5.18 (s) |
| A30 | $CH_3OCH_2CH_2-$ | H | H | O | 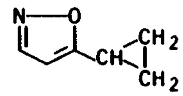 | 3 | |
| A31 | 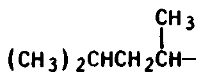 | H | H | O | 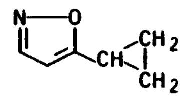 | 3 | |
| A32 | 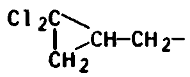 | H | H | O | 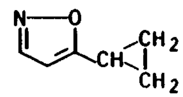 | 3 | |
| A33 | 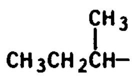 | H | H | O | 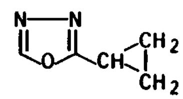 | 2 | 0.98 (t), 1.50-1.82 (m), 2.11-2.24 (m) |
| A34 | 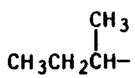 | H | H | S | 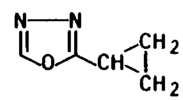 | 2 | |
| A35 | 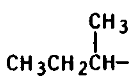 | H | H | $SO_2$ | 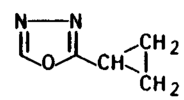 | 2 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B36 | 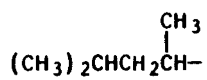 | H | H | O | 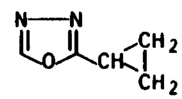 | 2 | |
| B37 | $(CH_3)_2CH-$ | H | H | O | 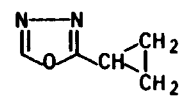 | 2 | |
| B38 | $CH_3OCH_2CH_2-$ | H | H | O | 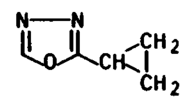 | 2 | |
| B39 | 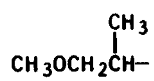 | H | H | O | 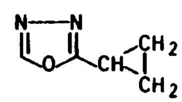 | 2 | |
| B40 | 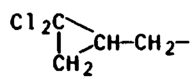 | H | H | O | 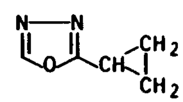 | 2 | |
| B41 | 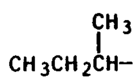 | H | H | O | 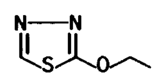 | 5 | 1.27 (d), 1.50 (t), 5.24 (s) |
| B42 | 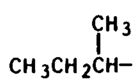 | H | H | S | 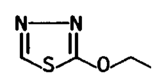 | 5 | 1.20 (d), 1.47 (t), 5.28 (s) |

| Nr. | $R^1$ | $R^2$ | Z | X | $Q_b$ | Substitution in Stellung ... von $Q_b$ | 250-MHz-$^1H$-NMR [ppm] in $CDCl_3$ (charakteristische Signale) |
|---|---|---|---|---|---|---|---|
| B43 | 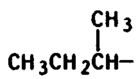 | H | H | $SO_2$ | 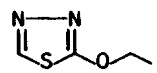 | 5 | |
| B44 | 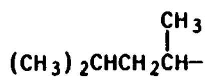 | H | H | O | 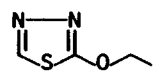 | 5 | 1.24 (d), 1.32 (t), 5.25 (s) |
| B45 | $(CH_3)_2CH-$ | H | H | O | 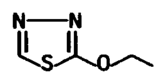 | 5 | 1.29 (d), 1.45 (t), 5.24 (s) |
| B46 | 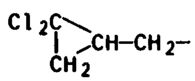 | H | H | O | 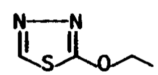 | 5 | 1.48 (t), 2,03-2.19 (m), 5.33 (s) |
| B47 | 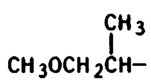 | H | H | O | 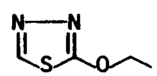 | 5 | 1.26 (d), 1.47 (t), 5.25 (s) |
| B48 | $CH_3OCH_2CH_2-$ | H | H | O | 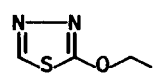 | 5 | 1.46 (t), 3.47 (s), 5.28 (s) |
| B49 | 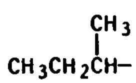 | H | H | O | 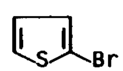 | 5 | 0.99 (t), 1.28 (d), 5.09 (s) |
| B50 | 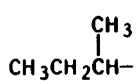 | H | H | S | 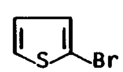 | 5 | 1.22 (d), 1.37-1.58 (m), 6.71-6.96 (m) |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B51 | 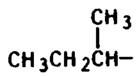 | H | H | $SO_2$ | 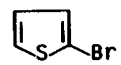 | 5 | |
| B52 | 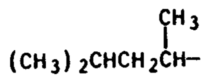 | H | H | O | 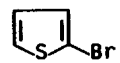 | 5 | 1.63-1.91 (m), 4.23-4.38 (m), 5.02 (s) |
| B53 | $(CH_3)_2CH-$ | H | H | O | 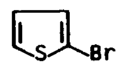 | 5 | 4.45-4.52 (m), 5.08 (s), 6.71-6.92 (m) |
| B54 | $CH_3OCH_2CH_2-$ | H | H | O | 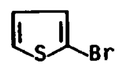 | 5 | 3.47 (s), 3.70-3.78 (m), 4.02-4.10 (m) |
| B55 | 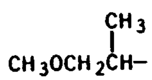 | H | H | O | 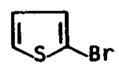 | 5 | 1.26 (d), 3.39 (s), 5.04 (s) |
| B56 | 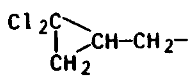 | H | H | O | 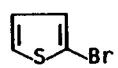 | 5 | 1.37 (t), 2.05-2.17 (m), 4.07 (d) |
| B57 | 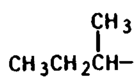 | H | H | O | 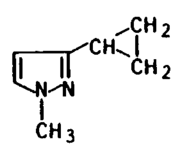 | 5 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B58 | 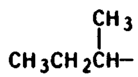 | H | H | S | 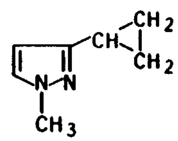 | 5 | |
| B59 | 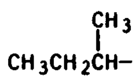 | H | H | O | 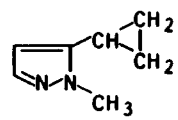 | 3 | 1.24 (d), 3.85 (s), 4.91 (s) |
| B60 | 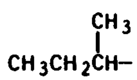 | H | H | S | 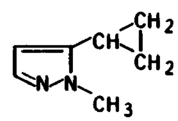 | 3 | 3.83 (s), 4.92 (s), 5.94 (s) |
| B61 | 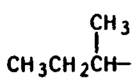 | H | H | $SO_2$ | 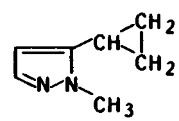 | 3 | |
| B62 | 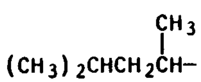 | H | H | O | 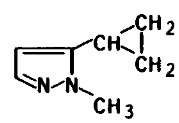 | 3 | 1.22 (d), 3.87 (s), 4.92 (s) |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B63 | 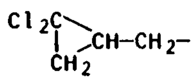 | H | H | O | 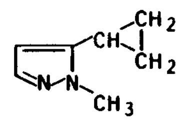 | 3 | 2.03-2.19 (m), 4.93 (s), 5.95 (s) |
| B64 | $CH_3OCH_2CH_2-$ | H | H | O | 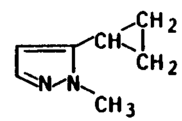 | 3 | 3.43 (s), 4.93 (s), 5.93 (s) |
| B65 | 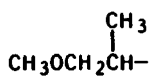 | H | H | O | 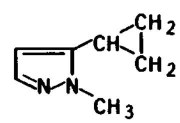 | 3 | 1.28 (d), 3.89 (s), 5.94 (s) |
| B66 | $(CH_3)_2CH-$ | H | H | O | 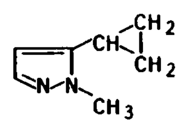 | 3 | 1.29 (d), 4.92 (s), 5.93 (s) |
| B67 | 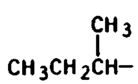 | H | H | O | 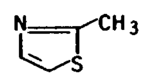 | 4 | |
| B68 | 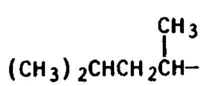 | H | H | O | 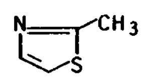 | 4 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B69 | 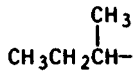 | H | H | O | 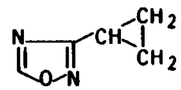 | 5 | 1.50-1.82 (m), 2.04-2.18 (m), 5.13 (s) |
| B70 | 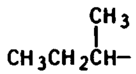 | H | H | S | 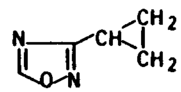 | 5 | |
| B71 | 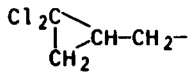 | H | H | O | 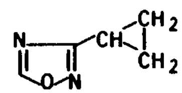 | 5 | |
| B72 | 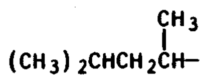 | H | H | O | 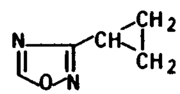 | 5 | |
| B73 | 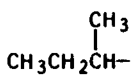 | H | H | O | 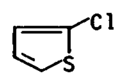 | 4 | 1.52-1.84 (m), 4.93 (s), 7.08 (s) |
| B74 | 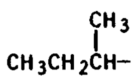 | H | H | O | 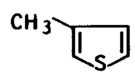 | 2 | 0.98 (t), 1.51-1.82 (m), 2.28 (s) |
| B75 | 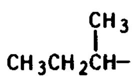 | H | H | O | 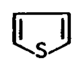 | 2 | 5.10 (s), 6.72-7.06 (m), 7.28-7.32 (m) |
| B76 | 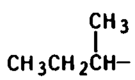 | H | H | O | 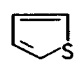 | 3 | 4.12-4.28 (m), 5.05 (s), 7.30-7.41 (m) |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B77 | $CH_3CH_2CH(CH_3)-$ | H | H | O | 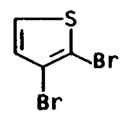 | 4 | 4.01-4.24 (m), 4.88 (s), 7.32 (s) |
| B78 | $CH_3CH_2CH(CH_3)-$ | H | H | O | 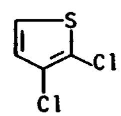 | 4 | 1.48-1.82 (m), 6.75-6.98 (m), 7.18 (s) |
| B79 | $CH_3CH_2CH(CH_3)-$ | H | H | O | 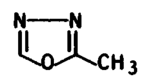 | 2 | 1.23 (d), 2.52 (s), 5.12 (s) |
| B80 | $CH_3CH_2CH(CH_3)-$ | H | H | O | 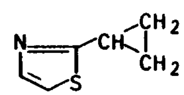 | 4 | |
| B81 | $CH_3CH_2CH(CH_3)-$ | H | H | S | 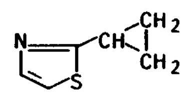 | 4 | |
| B82 | $(CH_3)_2CHCH_2CH(CH_3)-$ | H | H | O | 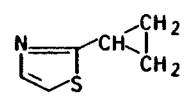 | 4 | |
| B83 | 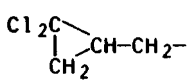 | H | H | O | 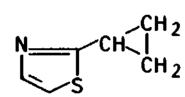 | 4 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B84 | $CH_3OCH_2CH_2-$ | H | H | O | 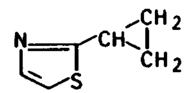 | 4 | |
| B85 | $CH_3$<br>$CH_3CH_2CH-$ | $CH_3$ | H | O | 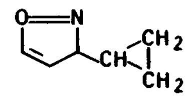 | 5 | |
| B86 | $CH_3$<br>$CH_3CH_2CH-$ | H | 2-F | O | 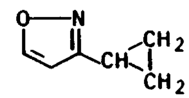 | 5 | |
| B87 | $CH_3$<br>$CH_3CH_2CH-$ | H | 2-F | O | 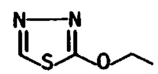 | 5 | |
| B88 | $CH_3$<br>$CH_3CH_2CH-$ | H | H | O | 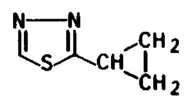 | 2 | 1.26 (d), 2.32-2.47 (m), 5.39 (s) |
| B89 | $CH_3$<br>$CH_3CH_2CH-$ | H | H | S | 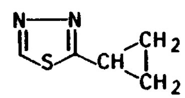 | 2 | 1.20 (d), 2.32-2.65 (m), 5.42 (s) |
| B90 | $CH_3$<br>$CH_3CH_2CH-$ | H | H | $SO_2$ | 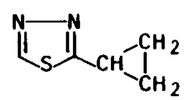 | 2 | |

| Nr. | $R^1$ | $R^2$ | Z | X | $Q_b$ | Substitution in Stellung ... von $Q_b$ | 250-MHz-$^1$H-NMR [ppm] in $CDCl_3$ (charakteristische Signale) |
|---|---|---|---|---|---|---|---|
| B91 | 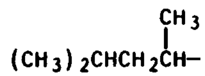 | H | H | O | 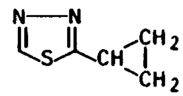 | 2 | 1.23 (d), 2.32-2.66 (m), 5.38 (s) |
| B92 | 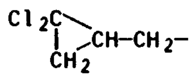 | H | H | O | 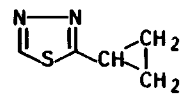 | 2 | 2.01-2.19 (m), 2.32-2.47 (m), 5.39 (s) |
| B93 | $CH_3OCH_2CH_2-$ | H | H | O | 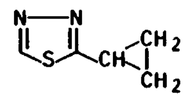 | 2 | 2.28-2.48 (m), 3.43 (s), 5.34 (s) |
| B94 | 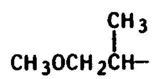 | H | H | O | 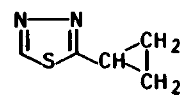 | 2 | 1.28 (d), 2.34-2.48 (m), 5.40 (s) |
| B95 | $(CH_3)_2CH-$ | H | H | O | 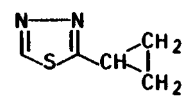 | 2 | 1.30 (d), 2.31-2.48 (m), 5.38 (s) |
| B96 | 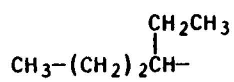 | H | H | O | 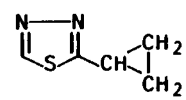 | 2 | 2.32-2.47 (m), 3.98-4.12 (m), 5.35 (s) |

Anwendungsbeispiele für N-phenoxymethylsubstituierte Azole Ia

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen. Die Reinheit der Substanzen wie der Vergleichsmittel lag bei > 95 %.

A: 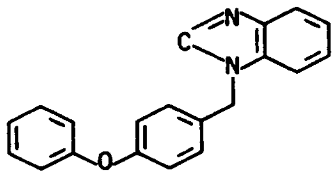

bekannt aus EP 132 606 als Verbindung Nr. 1

B: 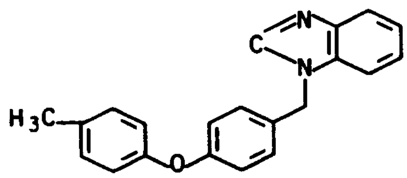

bekannt aus EP 132 606 als Verbindung Nr. 8

C: 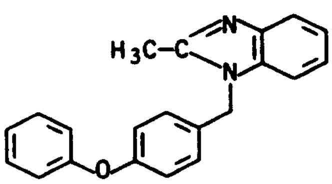

bekannt aus EP 132 606 als Verbindung Nr. 29

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens zwei Versuche angesetzt und ein Mittelwert gebildet.

Beispiel A.a

Dysdercus intermedius (Baumwollwanze), ovizide Wirkung

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt.

Die Eier werden für ca. 5 Sekunden in die wäßrige Wirkstoffaufbereitung getaucht, anschließend läßt man auf Filterpapier abtropfen. Dabei sollen die Eier nicht auf das Papier gelegt werden.

Die zurechtgeschnittenen Etiketten werden in Plastikpaletten gestellt, mit dem Klebestreifen nach oben. Eine halbe mit Wasser durchfeuchtete Watterolle wird in den Becher gelegt, um Austrocknung zu vermeiden und die Palette mit einer Glasplatte abgedeckt.

Nach ca. 8 Tagen wird bonitiert. (Bei der Kontrolle müssen die Larven geschlüpft sein).

Beurteilt wird die Schlupfhemmung in %.

Bei diesem Versuch erzielten die Verbindungen der Beispiele A1, A4, A20, A42 und A60 eine Schlupfhemmung von 80 % bei Wirkstoffkonzentrationen von 100 ppm und darunter, während mit den Vergleichsmitteln A, B und C bei einer Konzentration von 1000 ppm keine Schlupfhemmung beobachtet wurde.

Beispiel B.a

Dysdercus intermedius (Baumwollwanze), Zuchtversuch

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums.

Nach 24 Stunden überführt man die überlebenden Tiere in 1 l-Gläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen.

Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27 °C. Die Beobachtungszeit erstreckt sich bis zum Schlüpfen der Eier in den Kontrollen.

Bei diesem Versuch erzielten die Verbindungen der Beispiele A1, A4, A5, A20, A42 und A60 eine Mortalitätsrate von 100 % bei einer Wirkstoffkonzentration von 10 ppm und darunter, während die Vergleichsmittel bei einer Konzentration von 25 ppm praktisch unwirksam waren, d.h. eine Mortalitätsrate von weniger als 60 % bewirkten.

Anwendungsbeispiele für phenoxyalkylsubstituierte Fünfringheteroaromaten

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen:

D

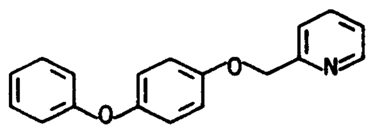

bekannt aus DE-A 25 16 331

E

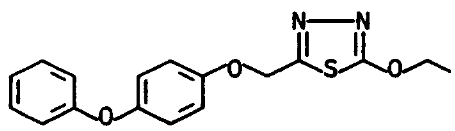

bekannt aus EP-A 239 047

Die Reinheit der Vergleichssubstanzen entsprach derjenigen der erfindungsgemäßen Verbindungen und lag bei mindestens 90 bis 95 %. Die Konzentrationen, bei denen die untersuchten Verbindungen eine 100 %ige Mortalität bzw. Hemmung bewirken, sind die jeweiligen Minimalkonzentrationen. Bei jeder Konzentration werden mindestens zwei Versuche durchgeführt.

Beispiel A.b

Dysdercus intermedius (Baumwollwanze), ovizide Wirkung

Stecketiketten wurden am oberen Rand mit Klebestreifenstücken (ca. 0,8 cm) beklebt. 24 Stunden vor Versuchsbeginn wurden die Eier durch Eintauchen in das "Sammelgefäß" an dem Klebestreifenrand befestigt.

Die Eier wurden für ca. 5 Sekunden in die wäßrige Wirkstoffaufbereitung getaucht, anschließend ließ man auf Filterpapier abtropfen. Dabei sollten die Eier nicht auf das Papier gelegt werden.

Die zurechtgeschnittenen Etiketten wurden in Plastikpaletten gestellt, Klebestreifen nach oben. Eine halbe mit Wasser durchfeuchtete Watterolle wurde in den Becher gelegt, um Austrocknung zu vermeiden und die Palette mit einer Glasplatte abgedeckt.

Nach ca. 8 Tagen wurde bonitiert. (Bei der Kontrolle müssen die Larven geschlüpft sein).

Beurteilt wurde die Schlupfhemmung in %.

Bei diesem Versuch wurde mit den Verbindungen B1, B4 und B7 eine Schlupfhemmung von 80 % bei Wirkstoffkonzentrationen von 400 ppm und darunter erzielt während die Vergleichsverbindung D bei 1000 ppm keine Schlupfhemmung bewirkte.

Beispiel B.b

Caenorhabditis elegans (Nematoden), Kontaktversuch

Auf die Oberfläche eines Nährbodens (5 ml in Kunststoffpetrischälchen Φ 35 mm, Höhe 10 mm) gab man 0,5 ml der acetonischen Wirkstofflösung. Nach Verdunsten des Acetons infizierte man mit 30 µl E-Coli-Bakterien und 50 µl Nematodensuspension. Nach 48 h ermittelte man die Kontaktwirkung in % Mortalität.

Bei einer Wirkstoffkonzentration von 100 ppm wurde mit den Verbindungen der Beispiele B2, B3, B4, B5 und B49 eine Mortalitätsrate von 100 % erzielt, während die Vergleichsmittel D und E bei dieser Konzentration praktisch unwirksam waren, d.h. eine Mortalitätsrate von weniger als 60 % aufwiesen.

## Patentansprüche

**1.** Phenoxyalkylsubstituierte Heteroaromaten der Formeln Ia und Ib

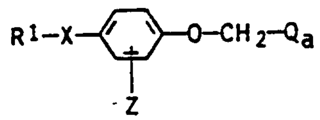

(Ia)

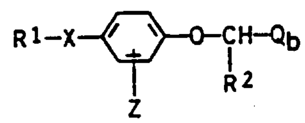

(Ib)

in denen die Substituenten die folgende Bedeutung haben:

| | |
|---|---|
| $R^1$ | $C_1$-$C_{12}$-Alkyl, $C_2$-$C_{12}$-Alkenyl, $C_2$-$C_{12}$-Alkinyl, $C_1$-$C_{12}$-Halogenalkyl, $C_2$-$C_{12}$-Halogenalkenyl, $C_2$-$C_{12}$-Halogenalkinyl, $C_3$-$C_{12}$-Alkoxyalkyl, $C_3$-$C_8$-Cycloalkyl, $C_4$-$C_{12}$-Cycloalkyl-alkyl, $C_3$-$C_8$-Halogencycloalkyl, $C_4$-$C_{12}$-Halogencycloalkyl-alkyl, $C_4$-$C_{12}$-Cycloalkyl-halogenalkyl oder $C_4$-$C_{12}$-Halogencycloalkyl-halogenalkyl; |
| X | O, S, $SO_2$; |
| Z | Wasserstoff, Halogen, $C_1$-$C_8$-Alkyl, $C_1$-$C_8$-Alkoxy, $C_1$-$C_4$-Halogenalkyl, $C_1$-$C_4$-Halogenalkoxy, $C_3$-$C_{10}$-Cycloalkyl, Nitro oder Cyano, im Fall des Heteroaromaten $Q_a$ und Wasserstoff, Halogen oder $C_1$-$C_4$-Alkyl im Fall des Heteroaromaten $Q_b$; |
| $R^2$ | Wasserstoff oder $C_1$-$C_4$-Alkyl; |
| $Q_a$ | ein gegebenenfalls substituierter Azolrest der Formel IIa-IIf |

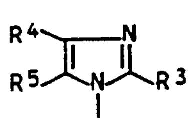

(IIa)

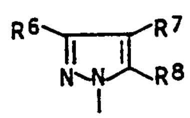

(IIb)

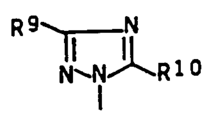

(IIc)

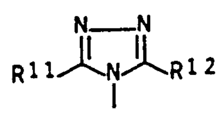

(IId)

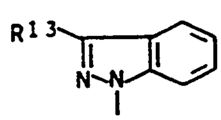

(IIe)

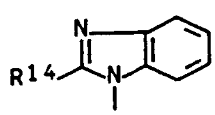

(IIf)

in denen

| | |
|---|---|
| $R^3$ bis $R^{14}$ | Wasserstoff, Halogen, $C_1$-$C_8$-Alkyl, $C_1$-$C_4$-Halogenalkyl, $C_1$-$C_4$-Alkoxy, $C_3$-$C_{10}$-Cycloalkyl, Phenyl, 1-Naphthyl und 2-Naphthyl, welches gegebenenfalls ein- bis dreifach durch Halogen, $C_1$-$C_8$-Alkoxy, $C_1$-$C_4$-Halogenalkyl oder $C_1$-$C_4$-Halogenalxoxy substituiert ist, |
| $Q_b$ | ein 5-Ring-Heteroaromat ausgewählt aus der Gruppe Thien-2--yl, Thien-3-yl, Thiazol-4-yl, Imidazol-5-yl, Pyrazol-3-yl, 1,3,4-Thiadiazol-2-yl, 1,3,4-Oxadiazol-2-yl, 1,2,4-Oxadiazol-3-yl, Isoxazol-5-yl und Isoxazol-3-yl, welcher gegebenenfalls ein- oder mehrfach mit Halogen, $C_1$-$C_8$-Alkyl, $C_2$-$C_8$--Alkenyl, $C_1$-$C_4$-Halogenalkyl, $C_1$-$C_8$-Alkoxy, $C_1$-$C_8$-Alkylthio, $C_2$-$C_8$-Alkoxyalkyl oder $C_3$-$C_{10}$-Cyclo- alkyl substituiert ist. |

**2.** Verfahren zur Herstellung der phenoxyalkylsubstituierten 5-Ring-Heteroaromaten der Formel I gemäß Anspruch 1, dadurch gekennzeichnet, daß man ein entsprechendes Phenol III

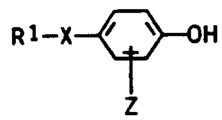

(III)

mit substituierten Heteroaromaten der Formeln IVa bzw. IVb

$Y\text{-}CH_2\text{-}Q_a$ (IVa)

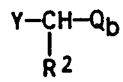

(IVb)

in denen Y für eine Abgangsgruppe steht, in Gegenwart einer Base oder das Phenolatanion von III direkt mit IVa bzw. IVb umsetzt.

**3.** Schädlingsbekämpfungsmittel, enthaltend einen phenoxyalkylsubstituierten 5-Ring-Heteroaromaten der Formel I gemäß Anspruch 1 neben hierfür üblichen Trägerstoffen.

**4.** Schädlingsbekämpfungsmittel gemäß Anspruch 3, enthaltend 0,1 bis 95 Gew.% eines phenoxyalkylsubstituierten 5-Ring-Heteroaromaten der Formel I gemäß Anspruch 1.

**5.** Verfahren zur Bekämpfung von Schädlingen, dadurch gekennzeichnet, daß man eine für Schädlinge wirksame Menge eines phenoxyalkylsubstituierten 5-Ring-Heteroaromaten der Formel I gemäß Anspruch 1 auf Schädlinge bzw. deren Lebensraum einwirken läßt.

## Claims

**1.** A phenoxyalkyl-substituted heteroaromatic of the formula Ia or Ib

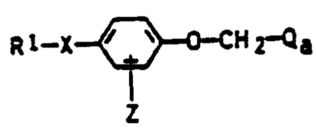

(Ia)

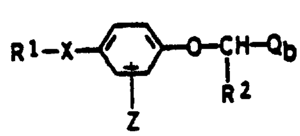

(Ib)

where $R^1$ is $C_1$-$C_{12}$-alkyl, $C_2$-$C_{12}$-alkenyl, $C_2$-$C_{12}$-alkynyl, $C_1$-$C_{12}$-haloalkyl, $C_2$-$C_{12}$-haloalkenyl, $C_2$-$C_{12}$-haloalkynyl, $C_3$-$C_{12}$-alkoxyalkyl, $C_3$-$C_8$-cycloalkyl, $C_4$-$C_{12}$-cycloalkylalkyl, $C_3$-$C_8$-halocycloalkyl, $C_4$-$C_{12}$-halocycloalkylalkyl, $C_4$-$C_{12}$-cycloalkylhaloalkyl or $C_4$-$C_{12}$-halocycloalkylhaloalkyl, X is O, S or $SO_2$, Z is hydrogen, halogen, $C_1$-$C_8$-alkyl, $C_1$-$C_8$-alkoxy, $C_1$-$C_4$-haloalkyl, $C_1$-$C_4$-haloalkoxy, $C_3$-$C_{10}$-cycloalkyl, nitro or cyano in the case of the heteroaromatic $Q_a$, and is hydrogen, halogen or $C_1$-$C_4$-alkyl in the case of the heteroaromatic $Q_b$, $R^2$ is hydrogen or $C_1$-$C_4$-alkyl, $Q_a$ is an unsubstituted or substituted azole radical of the formula IIa-IIf

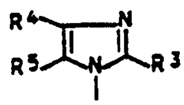
(IIa)

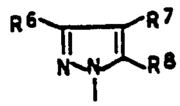
(IIb)

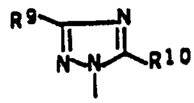
(IIc)

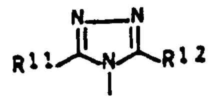
(IId)

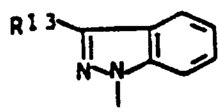
(IIe)

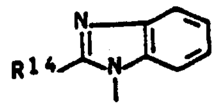
(IIf)

where $R^3$ to $R^{14}$ are each hydrogen, halogen, $C_1$-$C_8$-alkyl, $C_1$-$C_4$-haloalkyl, $C_1$-$C_4$-alkoxy, $C_3$-$C_{10}$-cycloalkyl, phenyl, 1-naphthyl or 2-naphthyl which is unsubstituted or monosubstituted to trisubstituted by halogen, $C_1$-$C_8$-alkoxy, $C_1$-$C_4$-haloalkyl or $C_1$-$C_4$-haloalkoxy and $Q_b$ is a heteroaromatic having a 5-membered ring, which is selected from the group consisting of thien-2-yl, thien-3-yl, thiazol-4-yl, imidazol-5-yl, pyrazol-3-yl, 1,3,4-thiadiazol-2-yl, 1,3,4-oxadiazol-2-yl, 1,2,4-oxadiazol-3-yl, isoxazol-5-yl and isoxazol-3-yl and is unsubstituted or monosubstituted or polysubstituted by halogen, $C_1$-$C_8$-alkyl, $C_2$-$C_8$-alkenyl, $C_1$-$C_4$-haloalkyl, $C_1$-$C_8$-alkoxy, $C_1$-$C_8$-alkylthio, $C_2$-$C_8$-alkoxyalkyl or $C_3$-$C_{10}$-cycloalkyl.

**2.** A process for the preparation of a phenoxyalkyl-substituted heteroaromatic having a 5-membered ring of the formula I as claimed in claim 1, wherein a corresponding phenol III

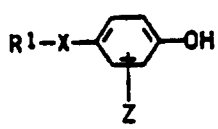
(III)

is reacted with a substituted heteroaromatic of the formula IVa or IVb

$Y$-$CH_2$-$Q_a$ (IVa)

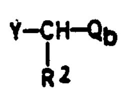
(IVb)

where Y is a leaving group, in the presence of a base, or the phenolate anion of III is reacted directly with IVa or IVb.

**3.** A pesticide containing a phenoxyalkyl-substituted heteroaromatic having a 5-membered ring of the formula I as claimed in claim 1, in addition to conventional carriers.

**4.** A pesticide as claimed in claim 3, containing from 0.1 to 95% by weight of a phenoxyalkyl-substituted heteroaromatic having a 5-membered ring of the formula I as claimed in claim 1.

**5.** A method for controlling pests, wherein a pesticidally effective amount of a phenoxyalkyl-substituted heteroaromatic having a 5-membered ring of the formula I as claimed in claim 1 is allowed to act on pests or their habitat.

## Revendications

**1.** Composés hétéroaromatiques à substituants phénoxyalkyle, de formules Ia et Ib

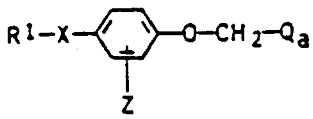

(Ia)

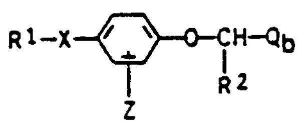

(Ib)

dans lesquelles les symboles ont les significations suivantes :

$R^1$ représente un groupe alkyle en C1-C12, alcényle en C2-C12, alcynyle en C2-C12, halogénoalkyle en C1-C12, halogénoalcényle en C2-C12, halogénoalcynyle en C2-C12, alcoxyalkyle en C3-C12, cycloalkyle en C3-C8, cycloalkyl-alkyle en C4-C12, halogénocycloalkyle en C3-C8, halogénocycloalkyl-alkyle en C4-C12, cycloalkyl-halogénoalkyle en C4-C12 ou halogénocycloalkyl-halogénoalkyle en C4-C12 ;

X représente O, S, $SO_2$ ;

Z représente l'hydrogène, un halogène, un groupe alkyle en C1-C8, alcoxy en C1-C8, halogénoalkyle en C1-C4, halogénoalcoxy en C1-C4, cycloalkyle en C3-C10, nitro ou cyano dans le cas du radical hétéroaromatique $Q_a$ et l'hydrogène, un halogène ou un groupe alkyle en C1-C4 dans le cas du radical hétéroaromatique $Q_b$ ;

$R^2$ représente l'hydrogène ou un groupe alkyle en C1-C4 ;

Qa représente un radical azolique éventuellement substitué de formule IIa à IIf

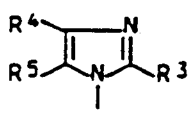

(IIa)

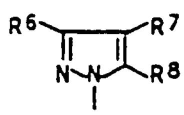

(IIb)

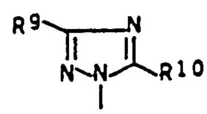

(IIc)

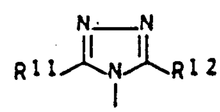

(IId)

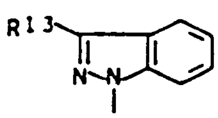

(IIe)

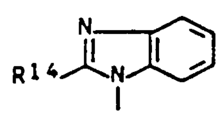

(IIf)

dans lesquelles

chacun des symboles $R^3$ à $R^{14}$ représente l'hydrogène, un halogène, un groupe alkyle en C1-C8, halogénoalkyle en C1-C4, alcoxy en C1-C4, cycloalkyle en C3-C10, phényl, 1-naphtyle ou 2-naphtyle portant éventuellement 1 à 3 substituants halogéno, alcoxy en C1-C8, halogénoalkyle en C1-C4 ou halogénoalcoxy en C1-C4,

$Q_b$ représente un radical hétéroaromatique à 5 chaînons choisi parmi les radicaux thiéno-2-yle, thiéno-3-yle, thiazole-4-yle, imidazole-5-yle, pyrazole-3-yle, 1,3,4-thiadiazole-2-yle, 1,3,4-oxadiazole-2-yle, 1,2,4-oxadiazole-3-yle, isoxazole-5-yle et isoxazole-3-yle et portant éventuellement un ou plusieurs substituants halogéno, alkyle en C1-C8, alcényle en C2-C8, halogénoalkyle en C1-C4, alcoxy en C1-C8, alkylthio en C1-C8, alcoxyalkyle en C2-C8 ou cycloalkyle en C3-C10.

**2.** Procédé de préparation des composés hétéroaromatiques à 5 chaînons, à substituants phénoxyalkyle, de formule I selon la revendication 1, caractérisé en ce que l'on fait réagir un phénol correspondant de formule III

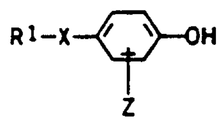 (III)

avec des composés hétéroaromatiques substitués de formules respectives IVa et IVb

$Y\text{-}CH_2Q_a$ (IVa)

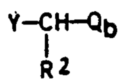 (IVb)

dans lesquelles Y représente un groupe éliminable, en présence d'une base, ou bien on fait réagir directement l'anion phénolate de III avec IVa ou IVb.

**3.** Produit parasiticide contenant un composé hétéroaromatique à 5 chaînons et à substituants phénoxyalkyle de formule I de la revendication 1, avec des véhicules usuels à cet effet.

**4.** Produit parasiticide selon la revendication 3, contenant 0,1 à 95 % en poids d'un composé hétéroaromatique à 5 chaînons et à substituants phénoxyalkyle de formule I de la revendication 1.

**5.** Procédé pour combattre les parasites, caractérisé en ce que l'on fait agir, sur les parasites ou leur habitat, une quantité parasiticide efficace d'un composé hétéroaromatique à 5 chaînons et à substituants phénoxyalkyle de formule I de la revendication 1.